# EUROPEAN PATENT SPECIFICATION

(11) **EP 3 555 780 B1**
(45) Date of publication and mention of the grant of the patent: **21.10.2020**
(21) Application number: 17816831.6
(22) Date of filing: 15.12.2017
(51) Int. Cl.: G16H 50/20

(54) **SYSTEM AND METHOD FOR DETERMINING AN IMPACT OF AN ACTIVE SUB-STANCE ON AN INFANT**
SYSTEM UND VERFAHREN ZUR BESTIMMUNG EINER WIRKUNG EINES WIRKSTOFFES AUF EIN KIND
SYSTÈME ET PROCÉDÉ POUR DÉTERMINER L'IMPACT D'UNE SUBSTANCE ACTIVE SUR UN NOURRISSON

(30) Priority: 16.12.2016 EP 16204597
(43) Date of publication of application: 23.10.2019
(73) Proprietor: Koninklijke Philips N.V., 5656 AG Eindhoven (NL)
(72) Inventor: BOURQUIN, Yannyk, Parulian, Julian, 5656 AE Eindhoven (NL); BROCKHUIS, Lili-Marjan, 5656 AE Eindhoven (NL); MENA BENITO, Maria, Estrella, 5656 AE Eindhoven (NL)
(74) Representative: Schudelaro, Antonius Adrianus Petrus
(86) International application number: PCT/EP2017/083126
(87) International publication number: WO 2018/109199

(56) References cited:
- WO-A1-2016/044368
- US-A1- 2003 144 883
- US-A1- 2007 077 168
- BIBIANA FRÃGULS ET AL: "A comprehensive review of assay methods to determine drugs in breast milk and the safety of breastfeeding when taking drugs", ANALYTICAL AND BIOANALYTICAL CHEMISTRY, SPRINGER, BERLIN, DE, vol. 397, no. 3, 13 April 2010 (2010-04-13), pages 1157-1179, XP019839219, ISSN: 1618-2650
- WILBECK J ET AL: "Pharmacologic Management of Acute Pain in Breastfeeding Women", JOURNAL OF EMERGENCY NURSING, C.V. MOSBY, ST. LOUIS, MO, US, vol. 34, no. 4, 1 August 2008 (2008-08-01) , pages 340-344, XP022929631, ISSN: 0099-1767, DOI: 10.1016/J.JEN.2007.07.006 [retrieved on 2008-05-07]

## Description

### FIELD OF THE INVENTION

The present invention relates to a system for determining an impact of an active substance on an infant, a corresponding method and computer program.

### BACKGROUND OF THE INVENTION

A consumption of active substances such as psychotropic drugs including caffeine, nicotine and alcohol, may affect an infant directly via the transmission of constituents in breast milk of a nursing mother.

Most active substances such as licit and illicit drugs consumed by a breastfeeding woman pass into the milk and can modify a production, volume and composition of the milk, as well as hypothetically have short- and long-term harmful effects on the infant. For instance, clinically significant toxicity in breast-fed infants from some substances used by mothers such as irritability, vomiting, sedation, respiratory depression, and sleep problems have been reported.

The article "A comprehensive review of assay methods to determine drugs in breast milk and the safety of breastfeeding when taking drugs" by B. Fräguls et al, Analytical And Bioanalytical Chemistry, Springer, Berlin, Vol. 397, No. 3, discloses analytical methods developed to detect different drugs in the breast milk, listing the principal characteristics and validation parameters, advantages and disadvantges, discusses the mechanisms of drug transfer into breast milk, describes the correlation between the concentration of the drug in breast milk and potential adverse outcomes on the infant for each drug, and indicates harm minimization strategies and approved breastfeeding recommendations.

It has therefore been an object of the present invention to provide a system, method and corresponding computer program which allow a determination of such impact of an active substance on an infant.

### SUMMARY OF THE INVENTION

The invention is defined by the appended claims. The invention comprises a system for determining an impact of an active substance on an infant according to claim 1. The system comprises a parameter providing unit for providing age and weight of the infant, an intake estimation unit for estimating the amount of intake of the active substance by a breastfeeding caregiver of the infant, a substance level determination unit for determining a level of the active substance in breast milk at the time of breastfeeding and/or milk extraction of the breastfeeding caregiver based on a history of the estimated intake of the active substance, an impact determination unit for determining an impact of the active substance on the infant based on i) the determined level of the active substance in the breast milk at the time of breastfeeding and/or milk extraction, and ii) the age and weight of the infant, and an interface unit for interacting with a user and/or with a further device delivering the active substance, wherein the interface unit is configured to, based on the determined impact of the active substance on the infant, at least one of i) provide an appropriate moment to breastfeed and/or initiate milk extraction, ii) provide an indication to limit intake of the active substance, and iii) initiate a change in operation of the further device.

Since the impact determination unit determines an impact of the active substance on the infant based on a level of the active substance in breast milk, which considers a history of estimated intake of the active substance by the breastfeeding caregiver, e.g. the infant's mother, the system according to the invention allows an accurate determination of an impact of the active substance on the infant based on, in substance, an intake of that substance by the breastfeeding caregiver. Since the impact determination unit further considers at least age and weight of the infant, the result of determination is further improved.

Preferentially, the term "infant" refers to a child that is at least to some extent fed with milk from a woman's breast. Since metabolism of infants drastically changes during the first months and years, considering the age allows for a more precise determination of the impact of the active substance of the infant. Similarly, the weight of the infant is advantageously considered since the active substances impact generally correlates with a concentration of the active substance per body weight.

The parameter providing unit can be a storing unit, on which age and weight and optionally further parameters of the infant are stored already. The parameter providing unit is then adapted to provide age and weight of the infant to the further units of the system. In this embodiment, the storing unit can be provided physically near the further units of the system or, in another embodiment, at a remote location, such as on a server. In further embodiments, the parameter providing unit can comprise an infant parameters measuring unit for measuring at least a weight of the infant and for providing the measured weight. Preferentially, the parameter providing unit can be configured to further provide parameters of the breastfeeding caregiver, such as her age and/or weight.

The intake estimation unit can comprise an input unit, for instance a smartphone display or a web server interface, into which a user, such as the breastfeeding caregiver itself, can input the amount of intake of an active substance preferentially together with a corresponding time of intake. In other embodiments, the intake estimation unit can also comprise a measuring unit including an optical camera, which is configured to estimate the amount of intake based on an evaluation of measured data, such as based on captured image data. In even other embodiments, the intake estimation unit can alternatively or additionally comprise other means for determining an intake of the active substance including a smart cup, a direct connection to a coffee machine, an electronic cigarette directly sending data regarding amount of nicotine consumed, without being limited to these examples.

The substance level determination unit preferentially determines the level at the time of breastfeeding based on the intake estimated by the intake estimation unit considering a time evolution of the concentration of the active substance in the breastfeeding caregiver. Further, preferentially the substance level determination unit considers a relation between the concentration of the active substance in the caregiver's body and in the breast milk provided by the breastfeeding caregiver, which can depend on the choice of the active substance.

Preferentially, the interface unit comprises a smartphone display, a tablet or a web interface accessible by any computer known in the art.

An appropriate moment to breastfeed and/or initiate milk extraction or an indication to limit intake of the active substance allows for limiting an impact on the infant, to improve milk production or to anticipate to a breastfeeding session. Preferentially, the interface unit can be configured for receiving an age and a weight of the infant. In other embodiments, also other data related to a user profile, such as data associated with the breastfeeding caregiver including her age and/or weight, can be stored on the system, a server, within an application, and so on, and be received and/or provided via the interface unit. Preferentially, the indication to limit intake of the active substance can be at least one of a quantity limitation and a time limitation.

Further devices delivering the active substance are preferentially, without being limited, devices that provide and/or prepare consumable products that include the active substance, such as coffee machines preparing coffee and electronic cigarettes providing nicotine containing smoke. Preferentially, a change in operation of said further device can include that the consumable product be provided with a smaller concentration or without the active substance. For instance, a coffee machine can be set to prepare decaffeinated coffee and/or an electronic cigarette can be switched into a nicotine-free mode.

In an embodiment the active substance comprises at least one psychotropic substance including caffeine, nicotine, alcohol, antidepressants, antipsychotics, antimaniac drugs, and anxiolytics.

Psychotropic substances as used herein should comprise all substances affecting mental activity, behaviour or perception, as well as mood altering substances such as all licit and illicit drugs. Preferentially, the active substance comprises at least one of caffeine, nicotine and alcohol, since caffeine, nicotine and alcohol are among the most popular active substances consumed by a large portion of the population. All of these active substances have shown to be present in breast milk and have an effect on infants.

The concentration of an active substance in breast milk thus depends on the dose of active substance ingested by the breastfeeding caregiver, a duration of the consumption, an amount of milk excreted daily and a general health of the breastfeeding caregiver.

For instance, several indices of nutritional importance have been shown to be affected by coffee and/or caffeine intake, which will now be discussed as an example for the active substance. However, it should be noted that similar affects of other active substances are known and can likewise be considered by the impact determination unit.

More precisely, it has been shown that zinc concentration in breast milk can be significantly depressed by coffee intake, coffee has been negatively related to infant iron (Fe) metabolism leading to decreased hemoglobin (Hb) and hematocrit (Hct) levels. As a result, infants of coffee consumers' as breastfeeding caregivers have a higher risk of anemia.

It has been shown that approximately 0.6% to 1.5% of the breastfeeding caregivers coffee intake is available to the infant through milk provided by a lactating woman. Though a rather small percentage of the caregiver's dose intake is, in the example of caffeine, transferred to the infant through breast milk, it can nevertheless accumulate because it cannot as easily be broken down by the infant and eliminated from the body, as by adults. For instance, the half-life of caffeine is about 4.9 hours in average for adults, wherein it can be up to 130 hours for newborns. Accordingly, newborn babies are particularly sensitive to caffeine. This information, in particular the dependency of the metabolizing rate on age, can be made available through the impact determination unit and will be considered by the impact determination unit in determining the impact on the infant. Infants until 3 months of age are unable to metabolize caffeine, which is only excreted in nature through urine. This decreases with a baby's age, for instance by 5 months of age the half-life of caffeine would already have been dropped to about 14 hours. Approximately at the age of 4 to 9 months, infants can arrive at the half-life time for adults, which is between 3 and 7 hours, more precisely approximately 4.9 hours on average. Of course is this data only exemplary data and other dependencies on age and weight, considering individual factors of the infant, for instance, are contemplated in other embodiments.

Likewise, other active substances such as nicotine and alcohol have been shown to be both present in breast milk and having affects/impact on infants. For instance, an acute episode of smoking by lactating women altered infants' sleep and wake patterning and the level of nicotine transferred into the breast milk is more than double the amount present in the plasma. Similarly, some of the alcohol consumed by a lactating woman is transferred to her breast milk which may adversely affect the infants sleep and gross motor development.

In an embodiment the impact determination unit is configured to estimate the impact of the active substance on the infant based on an amount of breast milk taken by the infant. The amount of breast milk taken by the infant can preferentially be estimated based on average values, provided by the parameter providing unit or determined by a milk intake determination device in communication with the system.

In an embodiment the impact determination unit is configured to estimate the amount of intake of the active substance by the infant based on i) the determined level of the active substance in the breast milk at the time of breastfeeding and/or milk extraction and ii) an amount of breast milk taken by the infant, and to determine the impact of the active substance on the infant from the estimated amount of intake and the age and weight of the infant.

In this embodiment, the impact on the infant is determined in a an two step approach, in a first step the amount of intake by the infant is determined and in a second step the impact of the active substance based on the estimated amount of intake is determined.

In an embodiment the impact determination unit is configured to track a level of the active substance in the infant based on i) the determined level of the active substance in the breast milk at the time of breastfeeding and/or milk extraction and ii) the amount of breast milk taken by the infant, and iii) a time of intake of the active substance by the infant.

A time of intake of the active substance of the infant can differ from the time of extracting the breast milk from the breastfeeding caregiver. In other examples, particularly in the case of breastfeeding, the time of intake corresponds to the time of milk extraction. No metabolism takes place after the time of milk extraction, therefore, the amount of active substance in the extracted breast milk will remain constant after extraction.

In an embodiment the user is the breastfeeding caregiver, and/or the further device delivering the active substance is at least one of a coffee machine and an electronic cigarette.

In an embodiment the appropriate moment to breastfeed is determined based on the determined level of the active substance in the breastfeeding caregiver and the impact of the active substance on the infant. Preferentially, at least one of the impact determination unit, the interface unit and a dedicated unit is configured to determine the appropriate moment to breastfeed.

Preferably, also a level of active substance in the infant as described with respect to further embodiments can be considered additionally or alternatively for determining the appropriate moment to breastfeed. Alternatively, instead of determining the appropriate moment to breastfeed, an appropriate moment for milk extraction can be determined.

In an embodiment the interface unit comprises a user input unit, preferably a smartphone, wherein the user input unit is configured to receive input indicative of an amount of intake of the active substance by the breastfeeding caregiver.

The amount of intake of the active substance can be provided to the user input unit as a quantity of the active substance itself, e.g. in milligrams or grams. In other examples, the amount of intake of the active substance can be provided by means of known consumable quantities, for instance, in terms of cups of coffee with respect to caffeine being the active substance, an amount of cigarettes corresponding to nicotine as the active substance and an amount of standard drinks concerning alcohol as the active substance. However, also other examples indicative of an amount of the respective active substance are of course contemplated for the mentioned or other active substances.

In an embodiment the system further comprises a monitoring unit for monitoring a sign of the infant, wherein the impact determination unit is configured to determine the impact of the active substance on the infant based on the monitored sign.

In this embodiment, an impact on the infant can be determined based on monitored signs and does not only rely on previously known or average data indicative of such impact. Accordingly, in this embodiment, the impact of the active substance on the infant can be determined more accurately. For instance, infants can become unhappy, jittery, colicky and/or sleep poorly as an affect of intake of active substances, which can be determined based on the monitored sign.

In an embodiment the monitoring unit includes at least one of a camera, a thermal camera and a microphone. In a further embodiment the sign includes at least one of a noise, a movement, a sleeping pattern, a crying activity and vital signs such as heart rate, breathing rate, body temperature and blood pressure. In a further embodiment the monitoring unit additionally or alternatively includes at least one contact monitoring unit for monitoring signs, preferentially vital signs, such as an electrocardiograph for conducting electrocardiography (ECG), a photoplethysmograph for conducting photoplethysmography (PPG) and a ballistocardiograph for conducting ballistocardiography (BCG).

These are of course only examples of suitable monitoring units and feasible signs, additional or alternative monitoring units and/or monitored signs are also contemplated.

In an embodiment the substance level determination unit is configured to determine an average level of the active substance in the breastfeeding caregiver for a predefined period of time, wherein the impact determination unit is configured to determine the impact of the active substance on the infant based on a correlation of the average level of the active substance and the monitored sign.

In this embodiment, the impact of the active substance on the infant can directly be measured by a correlation of an average level of the active substance to the infant's sign, which is monitored by the monitoring unit. Preferably, the predefined period of time corresponds to one day, wherein also longer or shorter periods can be considered. This embodiment is based on the assumption that breastfeeding and moments of intake of the active substance by the infant are distributed arbitrarily over the day leading to an average intake by the infant. Accordingly, no tracking on the concentration or level of the active substance in the infant is necessary in this embodiment, which makes it an easy and simple to use implementation which does not require extensive input. Preferentially, in case an impact on signs is detected by the correlation, the system can employ the user interface unit for providing an indication to limit intake of the active substance or modify the time between intake of the active substance and breastfeeding.

In an embodiment the system further comprises a milk intake determination device for determining the amount of breast milk taken by the infant, wherein the milk intake determination device comprises at least one of i) a bio-impedance measurement component, ii) a breast pump, iii) a strain gauge, iv) a breast volume measurement system, v) a nipple shield including a flow sensor, and vi) a feeding bottle.

Using a milk intake determination device, the amount of breast milk taken by the infant can be determined more accurately. In the alternative to using a milk intake determination device, an average or estimate of breast milk taken by the infant can be assumed, for instance based on known data.

Bioelectrical-impedance or short bio-impedance measurement is a commonly used method for estimating a body composition and can advantageously be employed for determining the amount of breast milk taken by the infant. Additionally or alternatively, a breast pump can be employed for determining this amount. The breast pump can comprise an indicator which allows a manual reading of the amount of breast milk or, additionally or alternatively, a component which measures or determines the amount of breast milk contained in the breast pump or a feeding bottle attached thereto. Additionally or alternatively, other milk intake determination not limited to the examples given above can be considered.

In an embodiment the milk intake determination device comprises a breast pump and/or a feeding bottle, wherein the breast pump and/or the feeding bottle comprises a communication unit, wherein the communication unit is configured to communicate at least one of a time and amount of the milk intake of the infant, in particular to at least one of the further units of the system.

The breast pump preferentially comprises a sensor for determining an inflow and/or an outflow of breast milk into or out of the breast pump., For instance, such sensor can include scales which measure the weight of a fluid container for containing the breast milk. Also other known sensors for determining an inflow and/or an outflow can of course be employed. The communication unit can in other embodiments also be configured to communicate with remote entities, such as a server, data base and so on. Preferentially, the breast pump comprises a separable feeding bottle, wherein the communication unit and/or the sensor can then be provided with the separable feeding bottle.

In an embodiment the system further comprises a sensor for determining an amount of the active substance in extracted breast milk, wherein the impact determination unit is configured to be calibrated based on the amount of the active substance determined by the sensor.

In an embodiment the sensor is arranged in at least one of a funnel, a valve and a feeding bottle of a breast pump. The breast pump in this embodiment can be a different breast pump or the same breast pump as employed in the milk intake determination device of the previous embodiments. Expressed differently, this embodiment can readily be combined with the milk intake determination device of the embodiment described above. The determination of the amount of active substance can be performed at different stages within the breast pump in accordance with the positioning of the sensor.

In this embodiment, the system therefore comprises a sensor which can measure the amount of the active substance in the breast milk such that the estimated amount or level of the active substance in the breast milk determined by the substance level determination unit can be calibrated based on the measured amount of the active substance in the breast milk.

The invention comprises a method for determining an impact of an active substance on an infant using a processing unit, according to claim 14. The method comprises a) providing, using a parameter providing unit, age and weight of the infant, b) estimating, using an intake estimation unit, the amount of intake of the active substance by a breastfeeding caregiver of the infant, c) determining, using a substance level determination unit, a level of the active substance in breast milk at the time of breastfeeding and/or milk extraction of the breastfeeding caregiver based on a history of the estimated intake of the active substance, d) determining, using an impact determination unit, an impact of the active substance on the infant based on i) the determined level of the active substance in the breast milk at the time of breastfeeding and/or milk extraction, and ii) the age and weight of the infant, and interacting, using an interface unit, with a user and/or with a further device based on the determined impact of the active substance on the infant, wherein the interaction is at least one of i) providing an appropriate moment to breastfeed and/or initiate milk extraction, ii) providing an indication to limit intake of the active substance, and iii) initiating a change in operation of the further device.

The invention also comprises a computer program for determining an impact of an active substance on an infant according to claim 15. The computer program comprising program code means for causing a system as defined in claim 1 to carry out the method as defined in claim 14, when the computer program is run on the system.

It shall be understood that the system of claim 1, the method of claim 14 and the computer program of claim 15 have similar and/or identical preferred embodiments, in particular, as defined in the dependent claims.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a system for determining an impact of an active substance on an infant,
Fig. 2 shows schematically and exemplarily a flowchart of using the system according to Fig. 1,
Fig. 3 shows schematically and exemplarily a further flowchart of using the system according to Fig. 1, and
Fig. 4 shows a flowchart exemplarily illustrating an embodiment of a method for determining an impact of an active substance on an infant.

### DETAILED DESCRIPTION OF EMBODIMENTS

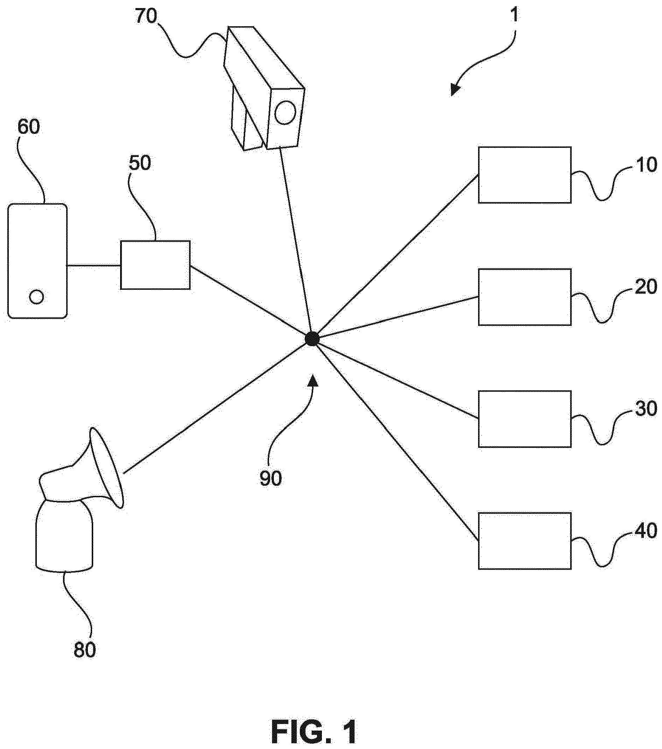

Fig. 1 shows schematically and exemplarily a system 1 for determining an impact of an active substance on an infant. The system 1 comprises a parameter providing unit 10, an intake estimation unit 20, a substance level determination unit 30, an impact determination unit 40, an interface unit 50, a user input unit 60, a monitoring unit 70 and a milk intake determination device 80. In the example of Fig. 1, all units of system 1 can communicate with each other via a symbolic hub 90.

Parameter providing unit 10 is configured to provide at least the age and a weight of the infant. Impact of the active substance significantly depends on age and weight of the infant, information which can be stored in parameter providing unit 10 itself, measured by a measuring unit comprised in parameter providing unit 10 or stored on a database, for instance within or associated with a user profile, and provided by parameter providing unit 10 from the database. In this example, parameter providing unit 10 is further configured to provide parameters related to the breastfeeding caregiver including at least one of her age and weight.

Intake estimation unit 20 is configured to estimate the amount of intake of the active substance by a breastfeeding caregiver of the infant, e.g. in most cases the infant's mother. The amount of intake can be estimated, for instance, using a user's input to interface unit 50. For instance, in case the active substance is caffeine, the user can input each time she consumes a cup of coffee, specifying details on the cup of coffee, such as type and size, and provide the time of consumption.

This information is taken by substance level determination unit 30, which is configured to determine a level of the active substance in breast milk at the time of milk expression. The time of milk expression can be the time of breastfeeding and/or a time of milk extraction using, for instance, a breast pump exemplarily depicted as comprised in milk intake determination device 80. Preferentially, substance level determination unit 30 determines the level further based on parameters of the breastfeeding caregiver, such as a weight, age, etc. Even further, substance level determination unit 30 preferentially determines the level of active substance based on a history of the estimated intake estimated by intake estimation unit 20.

Impact determination unit 40 is configured to determine an impact of the active substance on the infant based on multiple parameters. For instance, impact determination unit 40 can determine the impact based on the determined level of active substance determined by substance level determination unit 30, infant parameters provided by parameter providing unit 10 and/or an amount of breast milk taken by the infant as determined by milk intake determination device 80. Further, impact determination unit 40 can determine the impact based on signs monitored by monitoring unit 70.

Interface unit 50 is configured to interact with a user and/or a further device delivering the active substance. Interface unit 50 preferentially provides at least an appropriate moment to breastfeed and/or initiate milk extraction or an indication to limit intake of the active substance, for instance in case the determined level of active substance is relatively high or ready. Additionally or alternatively, interface unit 50 can initiate a change in operation of the further device, such as switching a coffee machine to decaffeinated coffee or setting an electronic cigarette into a nicotine-free mode. Interface unit 50 can comprise a display for providing information or an interface, which can be included in a web application and be accessible by well-known user interface devices.

In the example of Fig. 1, interface unit 50 comprises a user input unit 60, which is configured to receive input from the user. User input unit 60 is in this example drawn as a smartphone, but can of course be a different known input unit in other examples. User input unit 60 can receive input indicative of an amount of intake of the active substance by the breastfeeding caregiver, such as a quantity and type of cups of coffees consumed in case of caffeine as active substance. In further examples, user input unit 60 is further configured to receive parameters, such as age and weight of the infant or parameters related to the breastfeeding caregiver, such as also the age and weight.

Monitoring unit 70 is configured to monitor a sign of the infant and comprises in this example a camera, wherein in other examples different monitoring units, such as thermal cameras and/or microphones, can alternatively or additionally be used. The sign includes, without being limited, a noise, a movement, a sleeping pattern, a heart rate, a breathing rate and an a cry activity of the infant.

Finally, milk intake determination device 80 is configured to determine the amount of breast milk, which is taken by the infant. In this example, milk intake determination device 80 is illustrated as a breast pump, in other examples also different devices suitable for determining the amount of breast milk taken by the infant, such as components for carrying out bio-impedance measurements, can be used.

The connection via hub 90 is intended to express that each unit of system 1 can in principle communicate with any other unit of the system, while some connections are not necessary to be implemented in practice. Hub 90 is not necessarily a physical entity and is illustrated only schematically as a central node. Although all connections between two units are indicated as are linked via hub 90, also direct communication connections between two units of system 1 can be implemented additionally or alternatively. It must further be noted that some, several or all of the connections are wired or wireless connections, wherein one, more or all of the units can be provided in physical vicinity to each other, i.e. physically in the same device or as units of the same application running on the same device, or at different physical locations, such as at least in part on one or more servers.

More specifically, at least one of parameter providing unit 10, intake estimation unit 20, substance level determination unit 30 and impact determination unit 40 can be implemented as an application, which can be installed on a mobile phone, for instance. However, in other examples, one, more or all of the previous determination units can be implemented on a server and be accessed, for instance, via a web interface using a mobile phone, a portable and/or stationary computer device and the like.

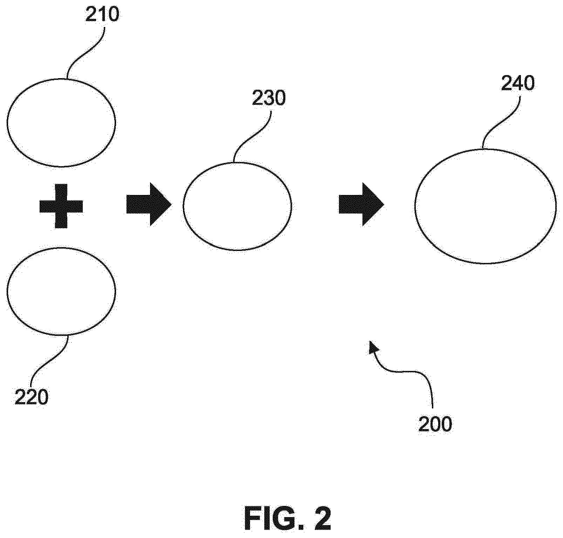

Fig. 2 shows schematically and exemplarily a flow chart 200 of using the system 1 according to Fig. 1. A first input 210 and a second input 220 are merged to evaluate in a step 230 an impact of active substances on an infant.

In this example, user interface unit 50 is provided with input, such as a drug intake, for instance, coffee, a weight of the mother and further parameters, such as lactation activity, to estimate the amount of intake and determine a level of the active substance in breast milk as first input 210.

The second input 220 relies on monitoring unit 70 and includes, for instance, a sleep pattern, a heart rate and/or a crying activity of the infant. Based on the input, motion and behaviour of the baby are measured.

In step 230, for instance by means of impact determination unit 40, the impact on the infant is estimated from inputs 210 and 220. Preferentially, parameters of the infant provided by parameter providing unit 10 are further employed in this step.

Finally, in step 240 the user is provided with a personalized advice, such as a counseling on intake of the drugs. The personal advice can include a most appropriate moment to breastfeed, to extract milk or to limit drug intake for limiting the impact on the baby and/or to improve milk production by anticipation to a breastfeeding session.

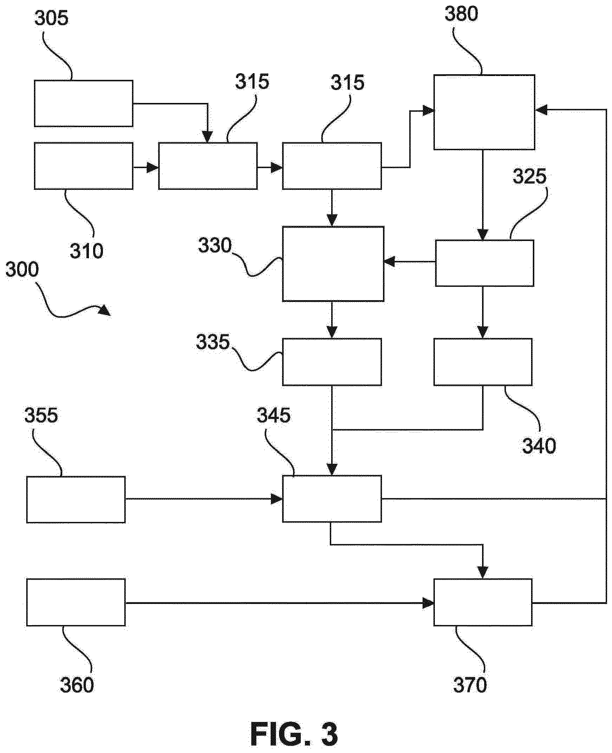

Fig. 3 shows a further flow chart of using a system 1 according to Fig. 1 schematically and exemplarily. Flow chart 300 is described with caffeine as an example for the active substance, wherein caffeine can be substituted by other active substances in other examples. Flow chart 300 illustrates the conditional and/or input based processing as interconnected nodes.

In node 305 parameters of the mother are provided and together with caffeine intake by the mother in node 310 provided to a determination node 315, in which the caffeine level in the caregiver's plasma is determined. Parameters of the mother include, for instance, age and weight and the caffeine intake can, for instance, be determined through user interface unit 50 and intake estimation unit 20.

Based on the caffeine level in plasma of node 315, a caffeine level in the breast milk is estimated in node 320. Based on the estimated caffeine level, and an occurring milk expression event in node 325, a caffeine level in the expressed milk and the milk volume is determined in node 330.

Based on the estimated caffeine level in node 330, the baby caffeine intake is estimated in node 335.

Based on the caffeine intake of node 335 and a feeding time in node 340, a caffeine level of the infant is estimated in node 345. The feeding time corresponds to the milk extraction time of node 325 in the case of breastfeeding, and corresponds to the time of feeding in the case of extracted milk, since the level of caffeine in the extracted milk will remain constant. In node 345, the caffeine level is estimated taking in account also a cumulative effect. Since the half-time of, for instance, caffeine highly depends on the age of the infant, baby parameters provided in node 350 are input to the caffeine level estimation in node 345. With node 360, a monitoring of signs of the infant is indicated. The estimated caffeine level and the monitoring signs are input to node 370, in which the signs are correlated with the estimated caffeine level. These information are then used for comparing the effect with an estimated effect of caffeine on the infant. Finally, in node 380, in this example a milk expression time and caffeine intake time/quantity advice for the mother is estimated using information from the caffeine level in the mother of node 320, the caffeine level in the baby of node 345 and the impact of caffeine on the baby derived from the correlation of node 370.

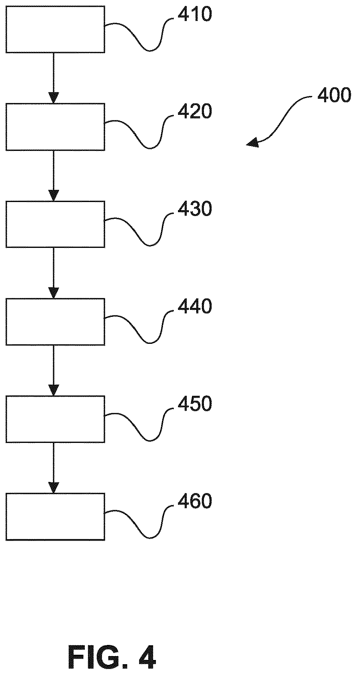

Fig. 4 shows a flow chart exemplarily illustrating an embodiment of a method 400 for determining an input of active substance on an infant.

In step 410, an age and weight of the infant is provided.

In step 420, the amount of intake of the active substance by a breastfeeding caregiver, such as the mother, of the infant is estimated.

In step 430, level of the active substance in breast milk at the time of breastfeeding and/or milk extraction is determined based on a history of the intake of the active substance estimated in step 420.

In step 440, an amount of breast milk taken by the infant is estimated, for instance by means of a milk intake determination device 80.

In step 450, an impact of the active substance on the infant is determined based on the level determined in step 430, the amount of breast milk taken by the infant determined in step 440, and the age and weight of the infant provided in step 410.

Finally, in step 460, an appropriate moment to breastfeed and/or initiate milk extraction or an indication to limit intake of the active substance is provided to user based on the determined impact of the active substance, determined in step 450, for instance by means of user interface unit 50.

A computer program may be stored/distributed on a suitable medium, such as an optical storage medium or a solid-state medium, supplied together with or as part of other hardware, but may also be distributed in other forms, such as via the Internet or other wired or wireless telecommunication systems including being downloadable or purchasable via an app store.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

## Claims

1. A system (1) for determining an impact of an active substance on an infant, comprising
- a parameter providing unit (10) for providing age and weight of the infant,
- an intake estimation unit (20) for estimating the amount of intake of the active substance by a breastfeeding caregiver of the infant,
- a substance level determination unit (30) for determining a level of the active substance in breast milk at the time of breastfeeding and/or milk extraction of the breastfeeding caregiver based on a history of the estimated intake of the active substance,
- an impact determination unit (40) for determining an impact of the active substance on the infant based on i) the determined level of the active substance in the breast milk at the time of breastfeeding and/or milk extraction, and ii) the age and weight of the infant, and
- an interface unit (50) for interacting with a user and/or with a further device delivering the active substance, wherein the interface unit (50) is configured to, based on the determined impact of the active substance on the infant, at least one of
i) provide an appropriate moment to breastfeed and/or initiate milk extraction,
ii) provide an indication to limit intake of the active substance, and
iii) initiate a change in operation of the further device.

2. The system according to claim 1, wherein the active substance comprises at least one psychotropic substance including caffeine, nicotine and alcohol.

3. The system according to claim 1, wherein the impact determination unit (40) is configured to estimate the amount of intake of the active substance by the infant based on i) the determined level of the active substance in the breast milk at the time of breastfeeding and/or milk extraction and ii) an amount of breast milk taken by the infant, and to determine the impact of the active substance on the infant from the estimated amount of intake and the age and weight of the infant.

4. The system according to claim 3, wherein the impact determination unit (40) is configured to track a level of the active substance in the infant based on
i) the determined level of the active substance in the breast milk at the time of breastfeeding and/or milk extraction and
ii) the amount of breast milk taken by the infant, and
iii) a time of intake of the active substance by the infant.

5. The system according to claim 1, wherein the user is the breastfeeding caregiver and/or the further device is at least one of a coffee machine and an electronic cigarette.

6. The system according to claim 1, wherein the interface unit (50) is configured to determine the appropriate moment to breastfeed based on the determined level of the active substance in the breastfeeding caregiver and the impact of the active substance on the infant.

7. The system according to claim 1, wherein the interface unit (50) comprises a user input unit (60), preferably a smartphone, and wherein the user input unit (60) is configured to receive input indicative of an amount of intake of the active substance by the breastfeeding caregiver.

8. The system according to claim 1, further comprising a monitoring unit (70) for monitoring a sign of the infant, wherein the impact determination unit is configured to determine the impact of the active substance on the infant based on the monitored sign.

9. The system according to claim 8, wherein the monitoring unit (70) includes at least one of a camera, a thermal camera, a microphone, an electrocardiograph, a photoplethysmograph and a ballistocardiograph, and/or the sign includes at least one of a noise, a movement, a sleeping pattern, a crying activity and a vital sign such as a heart rate, a breathing rate, a body temperature and a blood pressure.

10. The system according to claim 8, wherein the substance level determination unit (30) is configured to determine an average level of the active substance in the breastfeeding caregiver for a predefined period of time, and wherein the impact determination unit (40) is configured to determine the impact of the active substance on the infant based on a correlation of the average level of the active substance and the monitored sign.

11. The system according to claim 1, further comprising a milk intake determination device (80) for determining the amount of breast milk taken by the infant, wherein the milk intake determination device (80) comprises at least one of
- a bio-impedance measurement component,
- a breast pump,
- a strain gauge,
- a breast volume measurement system,
- a nipple shield including a flow sensor, and
- a feeding bottle.

12. The system according to claim 11, wherein the milk intake determination device (80) comprises a breast pump and/or a feeding bottle, wherein the breast pump and/or the feeding bottle comprises a communication unit, and wherein the communication unit is configured to communicate at least one of a time and amount of the milk intake of the infant, in particular to at least one of the further units of the system.

13. The system according to claim 1, further comprising a sensor for determining an amount of the active substance in extracted breast milk, wherein the impact determination unit (40) is configured to be calibrated based on the amount of the active substance determined by the sensor, and wherein the sensor is preferably arranged in at least one of a funnel, a valve and a feeding bottle of a breast pump (80).

14. A method for determining an impact of an active substance on an infant using a processing unit, comprising
- providing (410), using a parameter providing unit (10), age and weight of the infant,
- estimating (420), using an intake estimation unit (20), the amount of intake of the active substance by a breastfeeding caregiver of the infant,
- determining (430), using a substance level determination unit (30), a level of the active substance in breast milk at the time of breastfeeding and/or milk extraction of the breastfeeding caregiver based on a history of the estimated intake of the active substance,
- determining (450), using an impact determination unit (40) an impact of the active substance on the infant based on i) the determined level of the active substance in the breast milk at the time of breastfeeding and/or milk extraction, and ii) the age and weight of the infant,
- interacting, using an interface unit (50), with a user and/or with a further device based on the determined impact of the active substance on the infant, wherein the interaction is at least one of
i) providing an appropriate moment to breastfeed and/or initiate milk extraction,
ii) providing an indication to limit intake of the active substance, and
iii) initiating a change in operation of the further device.

15. A computer program for determining an impact of an active substance on an infant, the computer program comprising program code means for causing a system (1) as defined in claim 1 to carry out the method (400) as defined in claim 14, when the computer program is run on the system (1).

## Patentansprüche

1. System (1) zur Bestimmung der Auswirkung eines Wirkstoffs auf ein Kleinkind, umfassend:
- eine Parameterbereitstellungseinheit (10) zum Bereitstellen von Alter und Gewicht des Kleinkindes,
- eine Aufnahmeschätzeinheit (20) zum Schätzen der Aufnahmemenge des Wirkstoffs durch eine stillende Pflegekraft des Kleinkindes,
- eine Einheit zur Bestimmung eines Substanzgehalts (30) zur Bestimmung des Wirkstoffgehalts in der Muttermilch zum Zeitpunkt des Stillens und/oder der Milchextraktion der stillenden Pflegeperson auf der Grundlage einer Vorgeschichte der geschätzten Aufnahme des Wirkstoffs,
- eine Einheit zur Bestimmung einer Auswirkung (40) zur Bestimmung einer Auswirkung des Wirkstoffs auf das Kleinkind auf der Grundlage von i) dem ermittelten Wirkstoffgehalt in der Muttermilch zum Zeitpunkt des Stillens und/oder der Milchextraktion und ii) Alter und Gewicht des Kleinkindes und
- eine Schnittstelleneinheit (50) zur Interaktion mit einem Benutzer und/oder mit einer weiteren Vorrichtung, die den Wirkstoff abgibt, wobei die Schnittstelleneinheit (50) konfiguriert ist, um basierend auf der bestimmten Auswirkung des Wirkstoffs auf das Kleinkind mindestens eines der folgenden auszuführen:
i) Bereitstellen eines geeigneten Zeitpunkts zum Stillen und/oder Einleiten der Milchextraktion;
ii) Bereitstellen einer Indikation zur Begrenzung der Aufnahme des Wirkstoffs und
iii) Einleiten einer Betriebsänderung der weiteren Vorrichtung.

2. System nach Anspruch 1, wobei der Wirkstoff mindestens eine psychotrope Substanz umfasst, die Koffein, Nikotin und Alkohol enthält.

3. System nach Anspruch 1, wobei die Einheit zur Bestimmung einer Auswirkung (40) konfiguriert ist, um die Menge der Aufnahme des Wirkstoffs von dem Kleinkind zu schätzen, basierend auf i) dem bestimmten Wirkstoffgehalt in der Muttermilch des Stillens und/oder der Milchextraktion und ii) einer vom Kleinkind aufgenommene Muttermilchmenge, und zur Bestimmung der Auswirkung des Wirkstoffs auf den Kleinkind anhand der geschätzten Aufnahmemenge sowie des Alters und Gewichts des Kleinkindes.

4. System nach Anspruch 3, wobei die Einheit zur Bestimmung einer Auswirkung (40) konfiguriert ist, um einen Wirkstoffgehalt in dem Kleinkind zu verfolgen, basierend auf
i) dem bestimmten Wirkstoffgehalt in der Muttermilch zum Zeitpunkt des Stillens und/oder der Milchextraktion und
ii) der vom Kleinkind aufgenommene Muttermilchmenge und
iii) einer Zeit der Aufnahme des Wirkstoffs des Kleinkindes.

5. System nach Anspruch 1, wobei der Benutzer die stillende Pflegekraft ist, und/oder die weitere Vorrichtung mindestens eine Kaffeemaschine und eine elektronische Zigarette ist.

6. System nach Anspruch 1, wobei die Schnittstelleneinheit (50) konfiguriert ist, um den geeigneten Zeitpunkt zum Stillen basierend auf dem bestimmten Wirkstoffgehalt in der stillenden Pflegekraft und der Auswirkung des Wirkstoffs auf das Kleinkind zu bestimmen.

7. System nach Anspruch 1, wobei die Schnittstelleneinheit (50) eine Benutzereingabeeinheit (60), vorzugsweise ein Smartphone, umfasst, und wobei die Benutzereingabeeinheit (60) konfiguriert ist, um eine Eingabe zu empfangen, die eine Aufnahmemenge des Wirkstoffs durch die stillende Pflegekraft anzeigt.

8. System nach Anspruch 1, ferner umfassend eine Überwachungseinheit (70) zum Überwachen eines Zeichens des Kleinkindes, wobei die Einheit zur Bestimmung einer Auswirkung konfiguriert ist, um die Auswirkung des Wirkstoffs auf das Kleinkind basierend auf dem überwachten Zeichen zu bestimmen.

9. System nach Anspruch 8, wobei die Überwachungseinheit (70) mindestens eine Kamera, eine Wärmekamera, ein Mikrofon, einen Elektrokardiographen, einen Fotoplethysmographen und einen Ballistokardiograph umfasst, und/oder das Zeichen mindestens eines von einem Geräusch, einer Bewegung, einem Schlafmuster, einer Weinaktivität und einem Vitalzeichen wie Herzfrequenz, Atemfrequenz, Körpertemperatur und Blutdruck enthält.

10. System nach Anspruch 8, wobei die Einheit zur Bestimmung eines Substanzgehalts (30) konfiguriert ist, um einen Durchschnittsgehalt des Wirkstoffs in der stillenden Pflegekraft für einen vordefinierten Zeitraum zu bestimmen, und wobei die Einheit zur Bestimmung einer Auswirkung (40) konfiguriert ist, um die Auswirkung des Wirkstoffs auf das Kleinkind basierend auf einer Korrelation zwischen dem durchschnittlichen Wirkstoffgehalt und dem überwachten Zeichen zu bestimmen.

11. System nach Anspruch 1, ferner umfassend eine Vorrichtung zur Bestimmung der Milchaufnahme (80) zum Bestimmen der vom Kleinkind aufgenommenen Muttermilchmenge, wobei die Vorrichtung zur Bestimmung der Milchaufnahme (80) mindestens eines umfasst, von
- einer Komponente zur Messung der Bioimpedanz,
- einer Milchpumpe,
- einem Dehnungsmessstreifen,
- einem Brustvolumenmesssystem,
- einem Nippelschutz mit Durchflusssensor und
- einer Milchflasche.

12. System nach Anspruch 11, wobei die Vorrichtung zur Bestimmung der Milchaufnahme (80) eine Milchpumpe und/oder eine Milchflasche umfasst, wobei die Milchpumpe und/oder die Milchflasche eine Kommunikationseinheit umfassen, und wobei die Kommunikationseinheit konfiguriert ist, um mindestens eine Zeit und eine Menge der Milchaufnahme des Kleinkindes zu kommunizieren, insbesondere an mindestens eine der weiteren Einheiten des Systems.

13. System nach Anspruch 1, ferner umfassend einen Sensor zum Bestimmen einer Menge des Wirkstoffs in extrahierter Muttermilch, wobei die Einheit zur Bestimmung einer Auswirkung (40) konfiguriert ist, um basierend auf der vom Sensor bestimmten Menge des Wirkstoffs kalibriert zu werden, und wobei der Sensor vorzugsweise in mindestens einem von einem Trichter, einem Ventil und einer Milchflasche einer Milchpumpe (80) angeordnet ist.

14. Verfahren zum Bestimmen einer Auswirkung eines Wirkstoffs auf ein Kleinkind unter Verwendung einer Verarbeitungseinheit, umfassend
- Bereitstellen (410) des Alters und des Gewichts des Kleinkindes, unter Verwendung einer Parameterbereitstellungseinheit (10),
- Schätzen (420) der Aufnahmemenge des Wirkstoffs durch eine stillende Pflegekraft des Kleinkindes, unter Verwendung einer Aufnahmeschätzeinheit (20),
- Bestimmen (430), unter Verwendung einer Einheit zur Bestimmung eines Substanzgehalts (30), eines Wirkstoffgehalts in der Muttermilch zum Zeitpunkt des Stillens und/oder der Milchextraktion der stillenden Pflegeperson auf der Grundlage einer Vorgeschichte der geschätzten Aufnahme des Wirkstoffs,
- Bestimmen (450) unter Verwendung einer Einheit zur Bestimmung einer Auswirkung (40), einer Auswirkung des Wirkstoffs auf das Kleinkind auf der Grundlage von i) dem ermittelten Wirkstoffgehalt in der Muttermilch zum Zeitpunkt des Stillens und/oder der Milchextraktion und ii) Alter und Gewicht des Kleinkindes,
- Interagieren, unter Verwendung einer Schnittstelleneinheit (50), mit einem Benutzer und/oder mit einer weiteren Vorrichtung basierend auf der bestimmten Auswirkung des Wirkstoffs auf das Kleinkind, wobei die Interaktion mindestens eines der folgenden ist:
i) Bereitstellen eines geeigneten Zeitpunkts zum Stillen und/oder Einleiten der Milchextraktion;
ii) Bereitstellen einer Indikation zur Begrenzung der Aufnahme des Wirkstoffs und
iii) Einleiten einer Betriebsänderung der weiteren Vorrichtung.

15. Computerprogramm zum Bestimmen einer Auswirkung eines Wirkstoffs auf ein Kleinkind, wobei das Computerprogramm Programmcode-Mittel umfasst, um ein System (1) nach Anspruch 1 dazu zu veranlassen, das Verfahren (400) nach Anspruch 14 auszuführen, wenn das Computerprogramm auf dem System (1) ausgeführt wird.

## Revendications

1. Système (1) pour déterminer un impact d'une substance active sur un nourrisson, comprenant
- une unité de fourniture de paramètres (10) pour fournir l'âge et le poids du nourrisson,
- une unité d'estimation d'ingestion (20) pour estimer la quantité d'ingestion de la substance active par une personne qui allaite le nourrisson,
- une unité de détermination du niveau de la substance (30) pour déterminer un niveau de substance active dans le lait maternel au moment de l'allaitement et/ou de l'extraction du lait de la personne qui allaite sur la base d'un historique de l'ingestion estimée de la substance active,
- une unité de détermination d'impact (40) pour déterminer un impact de la substance active sur le nourrisson sur la base i) du niveau déterminé de la substance active dans le lait maternel au moment de l'allaitement et/ou de l'extraction du lait, et ii) de l'âge et du poids du nourrisson, et
- une unité d'interface (50) pour interagir avec un utilisateur et/ou avec un autre dispositif administrant la substance active, où l'unité d'interface (50) est configurée pour, sur la base de l'impact déterminé de la substance active sur le nourrisson, exécuter au moins l'une parmi les opérations suivantes
i) fournir un moment approprié pour allaiter et/ou initier l'extraction du lait,
ii) fournir une indication pour limiter l'ingestion de la substance active, et
iii) initier un changement de fonctionnement de l'autre dispositif.

2. Système selon la revendication 1, dans lequel la substance active comprend au moins une substance psychotrope incluant la caféine, la nicotine et l'alcool.

3. Système selon la revendication 1, dans lequel l'unité de détermination d'impact (40) est configurée pour estimer la quantité d'ingestion de la substance active par le nourrisson sur la base i) du niveau déterminé de la substance active dans le lait maternel au moment de l'allaitement et/ou de l'extraction du lait et ii) d'une quantité de lait maternel prise par le nourrisson, et pour déterminer l'impact de la substance active sur le nourrisson à partir de la quantité estimée d'ingestion et de l'âge et du poids du nourrisson.

4. Système selon la revendication 3, dans lequel l'unité de détermination d'impact (40) est configurée pour suivre un niveau de la substance active chez le nourrisson sur la base
i) du niveau déterminé de la substance active dans le lait maternel au moment de l'allaitement et/ou de l'extraction du lait et
ii) de la quantité de lait maternel prise par le nourrisson, et
iii) du moment d'ingestion de la substance active par le nourrisson.

5. Système selon la revendication 1, dans lequel l'utilisateur est la personne qui allaite et/ou l'autre dispositif est au moins l'une parmi une machine à café et une cigarette électronique.

6. Système selon la revendication 1, dans lequel l'unité d'interface (50) est configurée pour déterminer le moment approprié pour allaiter en fonction du niveau déterminé de la substance active chez la personne qui allaite et de l'impact de la substance active sur le nourrisson.

7. Système selon la revendication 1, dans lequel l'unité d'interface (50) comprend une unité d'entrée utilisateur (60), de préférence un smartphone, et dans lequel l'unité d'entrée utilisateur (60) est configurée pour recevoir une entrée indiquant une quantité d'ingestion de la substance active par la personne qui allaite.

8. Système selon la revendication 1, comprenant en outre une unité de surveillance (70) pour surveiller un signe du nourrisson, dans lequel l'unité de détermination d'impact est configurée pour déterminer l'impact de la substance active sur le nourrisson sur la base du signe surveillé.

9. Système selon la revendication 8, dans lequel l'unité de surveillance (70) comprend au moins l'un parmi une caméra, une caméra thermique, un microphone, un électrocardiographe, un photopléthysmographe et un ballistocardiographe, et/ou le signe comprend au moins l'un parmi un bruit, un mouvement, un rythme de sommeil, une activité de pleurs et un signe vital tel qu'un rythme cardiaque, un rythme respiratoire, une température corporelle et une pression sanguine.

10. Système selon la revendication 8, dans lequel l'unité de détermination du niveau de la substance (30) est configurée pour déterminer un niveau moyen de la substance active chez la personne qui allaite pendant une période de temps prédéfinie, et dans lequel l'unité de détermination d'impact (40) est configurée pour déterminer l'impact de la substance active sur le nourrisson sur la base d'une corrélation du niveau moyen de la substance active et du signe surveillé.

11. Système selon la revendication 1, comprenant en outre un dispositif de détermination de consommation de lait (80) pour déterminer la quantité de lait maternel prise par le nourrisson, dans lequel le dispositif de détermination de la consommation de lait (80) comprend au moins l'un parmi
- un composant de mesure de bio-impédance,
- un tire-lait,
- une jauge de contrainte,
- un système de mesure du volume mammaire,
- un protège-téton comprenant un capteur de flux, et
- un biberon.

12. Système selon la revendication 11, dans lequel le dispositif de détermination de la consommation de lait (80) comprend un tire-lait et/ou un biberon, où le tire-lait et/ou le biberon comprend une unité de communication, et où l'unité de communication est configurée pour communiquer au moins une durée et une quantité de la consommation de lait du nourrisson, en particulier à au moins l'une des autres unités du système.

13. Système selon la revendication 1, comprenant en outre un capteur pour déterminer une quantité de la substance active dans le lait maternel extrait, dans lequel l'unité de détermination d'impact (40) est configurée pour être étalonnée sur la base de la quantité de la substance active déterminée par le capteur, et dans lequel le capteur est de préférence disposé dans au moins l'un parmi un entonnoir, une valve et un biberon d'un tire-lait (80).

14. Procédé pour déterminer un impact d'une substance active sur un nourrisson en utilisant une unité de traitement, comprenant
- fournir (410), à l'aide d'une unité de paramétrage (10), l'âge et le poids du nourrisson,
- estimer (420), à l'aide d'une unité d'estimation d'apport (20), la quantité d'ingestion de la substance active par une personne qui allaite le nourrisson,
- déterminer (430), à l'aide d'une unité de détermination de niveau de substance (30), un niveau de la substance active dans le lait maternel au moment de l'allaitement et/ou de l'extraction du lait de la personne qui allaite sur la base d'un historique de l'ingestion estimée de la substance active,
- déterminer (450), à l'aide d'une unité de détermination d'impact (40), un impact de la substance active sur le nourrisson sur la base i) du niveau déterminé de la substance active dans le lait maternel au moment de l'allaitement et/ou de l'extraction du lait, et ii) de l'âge et du poids du nourrisson,
- interagir, à l'aide d'une unité d'interface (50), avec un utilisateur et/ou avec un autre dispositif sur la base de l'impact déterminé de la substance active sur le nourrisson, où l'interaction est au moins l'une parmi
i) fournir un moment approprié pour allaiter et/ou initier l'extraction du lait,
ii) fournir une indication pour limiter l'ingestion de la substance active, et
iii) initier un changement de fonctionnement de l'autre dispositif.

15. Programme informatique pour déterminer un impact d'une substance active sur un nourrisson, le programme informatique comprenant des moyens de code de programme pour amener un système (1) tel que défini dans la revendication 1 à exécuter le procédé (400) tel que défini dans la revendication 14, lorsque le programme informatique est exécuté sur le système (1).
